## Europäisches Patentamt

## European Patent Office

## Office européen des brevets

(11) Veröffentlichungsnummer: **0 016 406**
**B1**

# EUROPÄISCHE PATENTSCHRIFT

(45) Veröffentlichungstag der Patentschrift:
26.05.82

(51) Int. Cl.³: **C 07 C 11/02**, C 07 C 1/20 //
. B01J29/28

(21) Anmeldenummer: **80101259.2**

(22) Anmeldetag: **12.03.80**

(54) Verfahren zur Herstellung von Olefinen aus Rohmethanol.

(30) Priorität: **14.03.79 DE 2909927**

(43) Veröffentlichungstag der Anmeldung:
**01.10.80 Patentblatt 80/20**

(45) Bekanntmachung des Hinweises auf die Patenterteilung:
**26.05.82 Patentblatt 82/21**

(84) Benannte Vertragsstaaten:
**AT BE DE FR GB IT NL**

(56) Entgegenhaltungen:
**DE-A-2 442 240**
**US-A-4 025 572**
**US-A-4 066 714**
**US-A-4 083 889**

(73) Patentinhaber: **BASF Aktiengesellschaft,
Carl-Bosch-Strasse 38, D-6700 Ludwigshafen (DE)**

(72) Erfinder: **Marosi, Laszlo, Dr., Londoner Ring 11,
D-6700 Ludwigshafen (DE)**
Erfinder: **Stabenow, Joachim, Dr., Am Feldrain 22,
D-6940 Weinheim (DE)**
Erfinder: **Schwarzmann, Matthias, Dr.,
Carl-Bosch-Strasse 54, D-6703 Limburgerhof (DE)**

## Verfahren zur Herstellung von Olefinen aus Rohmethanol

In neuerer Zeit gewinnen Bemühungen, Methanol zur Herstellung von Olefinen zu verwenden, zunehmendes Interesse. Methanol lässt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff weiter beibehalten werden. In den vergangenen Jahren sind daher Verfahren entwickelt worden, die die Herstellung von Olefinen aus Methanol und/oder Dimethyläther zum Gegenstand haben. Ein solches Verfahren ist beispielsweise in der DE-OS 2 615 150 beschrieben. Als Katalysator wird dabei der Aluminosilikatzeolith ZSM-5 verwendet, der eigentlich ein Aromatisierungskatalysator ist. Die Umsetzung kann aber durch verschiedene Massnahmen, insbesondere durch die Verkürzung der Verweilzeit, in Richtung Olefinbildung gelenkt werden. Weitere die Olefinbildung begünstigende Parameter sind insbesondere die Verdünnung von Methanol bzw. Dimethyläther mit Inertgasen oder die Verdünnung des Katalysators mit Bindemitteln. Die Erfahrung zeigt, dass hohe Olefinausbeuten nur durch eine sehr starke Verdünnung von Methanol und/oder Dimethyläther mit Inertgasen zu erzielen sind. Das Verfahren arbeitet daher nicht wirtschaftlich. Andere bekanntgewordene Verfahren haben als Nachteil eine geringe Belastbarkeit und eine schnelle Verkohlung des Katalysators. Es besteht daher grosses Interesse an einem einfachen Verfahren, das die vollständige Umsetzung von Rohmethanol und/oder Dimethyläther in überwiegend aus $C_2$-$C_5$-Olefinen bestehende Kohlenwasserstoffe ermöglicht.

Es wurde nun gefunden, dass man in einfacher Weise $C_2$-$C_5$-Olefine aus Rohmethanol und/oder Dimethyläther durch katalytische Umwandlung bei erhöhter Temperatur in Gegenwart von zeolithhaltigen Katalysatoren erhält, wenn man als Ausgangsstoff unverdünntes Rohmethanol und/oder unverdünnten Dimethylether und solche zeolithhaltige Katalysatoren, deren Zeolithanteil mit Hilfe von Hexamethylendiamin aus technischem Wasserglas ohne weitere Zugabe eines Metallsalzes hergestellt wurde, verwendet und deren Belastung im Bereich von 2 bis 50 g/g Zeolith.h einstellt.

Aus US-PS 4 025 572 und US-PS 4 066 714 ist auch schon die Verwendung von ähnlichen Katalysatoren mit Molverhältnissen von $SiO_2/Al_2O_3$ von 8.8 bis 200 beschrieben, die ein abweichendes katalytisches Verhalten zeigen, die sich vom Strukturtyp des ZSM-35 ableiten und in Gegenwart von Ethylendiamin hergestellt worden sind.

Eine bevorzugte Ausführungsform besteht darin, Rohmethanol bei einem Druck zwischen Normaldruck und etwa 30 bar und bei Temperaturen zwischen 300 und 700°C, bevorzugt bei 400 bis 650°C, an dem erfindungsgemässen Zeolithkatalysator umzusetzen. Unter Rohmethanol wird Methanol mit einem Wassergehalt bis etwa 30 Gew.-% verstanden, wie es in der Methanolsynthese entsteht. Die Belastung des Katalysators, ausgedrückt in g Methanol und/oder Dimethyläther/ g Kat.h, wird vorteilhafterweise so gewählt, dass diese Stoffe möglichst vollständig umgesetzt werden, damit keine Abtrenn- und Rückführprobleme entstehen. Die Belastung liegt daher in dem Bereich von 2 bis 50, vorzugsweise 5 bis 15 g/g Zeolith.h. Es ist ein besonderer Vorteil der Erfindung, dass man die Umsetzung von Rohmethanol bzw. Dimethyläther in $C_2$-$C_5$-Olefine ohne Verdünnungsmittel ausführen kann.

Als Zeolithkatalysatoren kann man die in der deutschen Patentanmeldung P 2 831 334.0 beschriebenen Zeolithe verwenden. Diese Katalysatoren zeichnen sich durch eine besonders hohe Aktivität und Selektivität aus.

Die Durchführung des erfindungsgemässen Verfahrens wird anhand der nachstehenden Beispiele näher erläutert.

Beispiel
Herstellung des Zeolithen:
Es werden drei Lösungen hergestellt. Lösung 1 besteht aus 326,6 g technischem Wasserglas (8 Gew.-%- $Na_2O$- und 28 Gew.-%- $SiO_2$-Gehalt) und 352 g Wasser.

Lösung 2 besteht aus 300 g einer 50prozentigen wässrigen Hexamethylendiaminlösung und Lösung 3 aus 508,3 g Wasser und 24,7 g 96prozentiger Schwefelsäure. Die Lösungen 2 und 3 werden nacheinander zu Lösung 1 unter Rühren hinzugegeben. Die erhaltene Mischung wird in einem Stahlautoklaven 5 Tage unter ihrem Eigendruck auf 150°C erhitzt. Das erhaltene Produkt wird filtriert, gewaschen und bei 100°C getrocknet.

Herstellung des Katalysators aus dem Zeolithen:
Der erhaltene Zeolith wird in einem solchen Mengenverhältnis mit Böhmit vermischt, dass der Zeolithgehalt in der Mischung, bezogen auf Wasser und aminfreie Basis, etwa 65 Gew.-% beträgt. Die Mischung wird anschliessend mit Wasser geknetet und zu Strängen mit 1 mm Durchmesser verstrangt. Die erhaltenen Stränge werden bei 540°C kalziniert und anschliessend bei 80°C mit einer wässrigen Ammoniumsulfatlösung behandelt, filtriert, gewaschen und getrocknet.

Umsetzung von Rohmethanol zu Olefinen:
20 g (Trockenbasis) dieses Katalysators werden in einen Durchflussreaktor mit 20 mm Durchmesser eingebaut und die Aktivität bei der Umsetzung von Rohmethanol in Olefine getestet. Die Reaktionsbedingungen und die Versuchsergebnisse sind in der folgenden Tabelle dargestellt. Die Reaktionsprodukte wurden gaschromatographisch analysiert.

| Eingangstemperatur | 400°C |
|---|---|
| Temperaturanstieg | 200°C |
| Druck | 1,3 bar |
| Belastung | 170 g Rohmethanol/h |
| Gesamtbelastung | 1000 g |
| Umsetzung | 95-100% |

Man erhält ein Reaktionsprodukt mit folgender Zusammensetzung:
Flüssige Kohlenwasserstoffe 18 Gew.-%, bezogen auf eingesetztes $CH_2$
Gasförmige Kohlenwasserstoffe 82 Gew.-%, bezogen auf eingesetztes $CH_2$
davon sind:

| Olefine | $C_2$ | 20 Vol.% |
|---|---|---|
| | $C_3$ | 43 » |
| | $C_4$ | 16 » |
| | $C_5$ | 5 » |
| Paraffine | $C_1$ | 4 » |
| | $C_2$ | Spuren |
| | $C_3$ | 2 Vol.% |
| | $C_4$ | 10 » |

## Patentanspruch

Verfahren zur Herstellung von $C_2$-$C_5$-Olefinen aus Rohmethanol und/oder Dimethylether durch katalytische Umwandlung bei erhöhter Temperatur in Gegenwart von zeolithhaltigen Katalysatoren, dadurch gekennzeichnet, dass man als Ausgangsstoff unverdünntes Rohmethanol und/oder unverdünnten Dimethylether und solche zeolithhaltige Katalysatoren, deren Zeolithanteil mit Hilfe von Hexamethylendiamin aus technischem Wasserglas ohne weitere Zugabe eines Metallsalzes hergestellt wurde, verwendet und dass man die Belastung im Bereich von 2 bis 50 g/g Zeolith.h einstellt.

## Claim

A process for the preparation of $C_2$-$C_5$-olefins from crude methanol and/or dimethyl ether by catalytic conversion at an elevated temperature in the presence of a zeolite-containing catalyst, characterized in that undiluted crude methanol and/or undiluted dimethyl ether is used as the starting material, and those zeolite-containing catalysts are used, the zeolite proportion of which has been prepared from technical-grade waterglass with the aid of hexamethylenediamine, without addition of a metal salt, and that the throughput is adjusted to from 2 to 50 g/g of zeolite/hr.

## Revendication

Procédé de préparation d'oléfines en $C_2$-$C_5$ à partir du méthanol brut et/ou de l'éther diméthylique par conversion catalytique à température élevée en présence de catalyseurs contenant des zéolithes, caractérisé en ce que l'on utilise comme produit de départ du méthanol brut non dilué et/ou de l'éther diméthylique non dilué et des catalyseurs contenant des zéolithes dont la fraction zéolithe a été préparée à l'aide de l'hexaméthylène-diamine à partir de la lessive industrielle de silicate de sodium sans autre adjonction d'un sel métallique, et en ce que l'on règle la charge dans l'intervalle de 2 à 50 g/g de zéolithe.h.